# EUROPEAN PATENT SPECIFICATION

(11) **EP 0 194 838 B1**
(45) Date of publication and mention of the grant of the patent: **09.06.1993**
(21) Application number: 86301700.0
(22) Date of filing: 10.03.1986
(51) Int. Cl.: A61K 9/16, A61K 9/22, A61K 31/18

(54) **Controlled-release pharmaceutical formulation**
Pharmazeutische Darreichungsform mit verzögerter Abgabe
Formulation pharmaceutique à libération contrôlée

(30) Priority: 08.03.1985 JP 46180/85
(43) Date of publication of application: 17.09.1986
(60) Divisional application: 92203126.5
(73) Proprietor: YAMANOUCHI PHARMACEUTICAL CO., LTD., Tokyo 103 (JP)
(72) Inventor: Fukui, Muneo, Urawa-shi Saitama (JP); Tomuro, Kouji, Shizuoka-shi Shizuoka (JP); Masuyama, Shigeru, Yaizu-shi Shizuoka (JP); Kajiyama, Atsushi 703-3, Kitamotodanchi, Kitamoto-shi Saitama (JP); Hikosaka, Tamio, Ageo-shi Saitama (JP); Aruga, Masayoshi, Ageo-shi Saitama (JP); Soeishi, Yoshiaki, Itabashi-ku Tokyo (JP); Higuchi, Saburo, Hasuda-shi Saitama (JP)
(74) Representative: Geering, Keith Edwin

(56) References cited:
- EP-A- 0 034 432
- EP-A- 0 187 703
- DD-A- 104 027
- DE-A- 1 467 781
- DE-A- 1 962 016
- DE-A- 2 148 391
- DE-A- 2 921 931
- GB-A- 630 439
- GB-A- 2 075 839
- US-A- 4 489 026
- PATENT ABSTRACTS OF JAPAN, unexamined applications, C field, vol. 9, no. 160, July 4, 1985 THE PATENT OFFICE JAPANESE GOVERNMENT page 144 C 289

## Description

This invention relates to a novel and effective pharmaceutical controlled-release formulation for oral administration.

When a controlled-release pharmaceutical formulation is administered to a living body, intra- or inter-individual variations in effect occur frequently influenced by factors in the pharmaceutical formulation or factors in the living body. One of the factors in the living body is a variation in gastrointestinal transit time. A formulation which reduces the effect of this factor is known (H.Bechgaad and G.H.Nielsen, Drug Devel.Ind.Pharm., 4, 53[1978]). This is a formulation form (such as tablets, hard capsules, etc.) which disintegrates in the gastrointestinal tract to form a number of particles (e.g., microcapsules, microspheres, etc.,) which are distributed broadly in the gastrointestinal tract and from which an active substance is gradually released.

Hitherto, there are known various materials and various production processes for obtaining individual particles (e.g.,microcapsules, microspheres etc.,) of controlled-release pharmaceutical formulations containing an active substance.

For example, waxes, lipids, water-insoluble macromolecular materials, ion-exchange resins, etc., are known as the above-mentioned materials. A production process for individual particles frequently requires a complicated and long step of preparing granules containing an active substance and other material(s) and applying thereto an enteric coating. In such a production process the production cost is frequently high and the reproducibility of the dissolving characteristics of products is not good.

As a material for forming a structured particle that is not easily disintegrated in the gastrointestinal tract, crystalline cellulose (formerly "microcrystalline cellulose") is known. A pharmaceutical formulation using crystalline cellulose in an amount of about 10 to 40% by weight based on the weight of the formulation is described in Japanese Patent publication No.5275/70. The above-described patent describes a controlled-release pharmaceutical formulation containing as active substance bis(o-benzoylthiamine)disulfide, but an enteric coating is necessary in order to prolong the release time. The pharmaceutical formulation of the patent has a structure which does not easily disintegrate in the gastrointestinal tract. It is known that if the amount of crystalline cellulose is about 10 to 40% by weight, the pharmaceutical formulation is insufficiently strong and also generally provide insufficient controlled-release of active substance. GB-A-1561204 discloses a sustained release formulation which is a hard gelatin capsule containing spheroids made of propranolol or salt thereof and crystalline cellulose alone, the spheroids being exemplified as made by mixing these components with water and then extruding, spheronising and drying; to give the sustained release formulation, these spheroids have to be film-coated before encapsulation.

Furthermore, European Patent Publication 80341A describes an invention of "oral pharmaceutical controlled release multiple-units formulation". However, in the invention, "cores" are produced by a complicated process and also enteric coating is applied thereto for obtaining controlled-release.

Moreover, the above-described pharmaceutical formulation does not disintegrate in the stomach and is prepared with the addition of disintegrants so that the coating is eroded and the core itself disintegrates in the small intestine.

DD-A-104027 discloses granulating mixtures of active substance, incompressible filler, and organic solvent solution of organic binder; the only fillers exemplified are inorganic materials; it is mentioned that cellulose may be used as a filler, and the requirement for low compressibility means that this would be non-crystalline. GB-A-2075839 discloses sustained release granules made by granulating mixtures of active substance, long chain fatty acid metal salt, crystalline cellulose diluent, and aqueous solution of water-soluble binder or organic solvent solution of water-insoluble binder. DE-A-3325652 discloses granulating a mixture including active substance, inorganic filler, crystalline cellulose and a solution of organic polymer in organic solvent, and then tabletting the granules with other ingredients; in other embodiments the ingredients are tabletted directly; the disclosed products are not indicated to be sustained release formulations. Chemical Abstracts, 1980, 93, 13003e discloses direct tabletting, without previous granulating, of a mixture of active substance, starch, crystalline cellulose and Mg stearate.

The present invention providces a method for making granules for a controlled-release pharmaceutical formulation, the method comprising granulating, to give granules of a diameter of from 0.1 to 1.5 mm, a mixture of (a) 5-{2-[2-(o-ethyoxyphenoxy)ethylamino]propyl}-2-methoxybenzene-sulfonamide hydrochloride, (b) crystalline cellulose carrier in an amount of at least 50 solids wt.% of the mixture and (c) release control agent comprising water and water-insoluble macromolecular substance, the release control agent being in the form of an aqueous emulsion or suspension or gel of said macromolecular substance or in the form of a solution of said macromolecular substance in an aqueous organic solvent, and said macromolecular substance being selected from acrylic acid series polymers and copolymers and cellulose derivatives, the amount of the release controlling agent being up to 30% (as solid component) or 50-150% (as aqueous liquid material) based on the weight of the granules. It also proovides a pharmaceutical sustained release formulation comprising granules of a diameter of from 0.1 to 1.5 mm obtainable by adding release control agent comprising water and water-insoluble macromolecular substance to a mixture of 5-{2-[2-(o-ethyoxyphenoxy)-ethylamino]propyl}-2-methoxybenzene-sulfonamide hydrochloride and crystalline cellulose carrier and granulating the resultant mixture, the crystalline cellulose carrier constituting at least 50% by weight of the solids in the mixture and the composition and amount of the release control agent being as defined for (c) above.

The controlled-release pharmaceutical formulation of the present invention is suitable for oral administration, can show a reproducible dissolution rate without enteric coating, and can be simply produced.

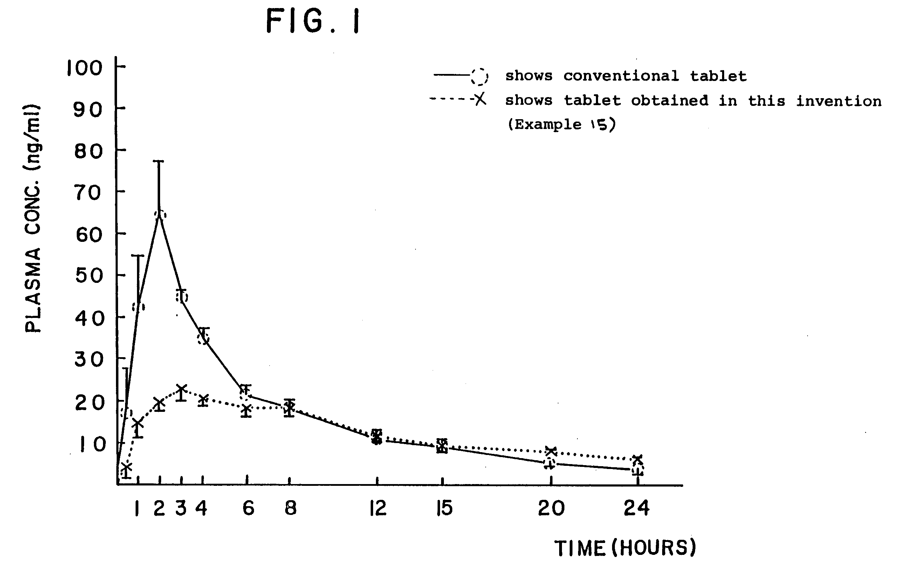

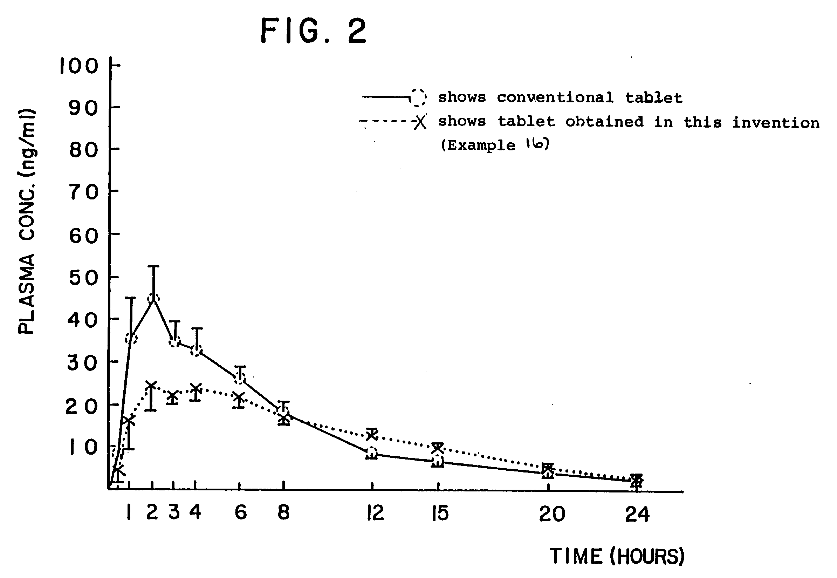

Fig. 1 and Fig. 2 are graphs each showing the change with the passage of time of the concentration of a physiologically active substance (5-{2-[2-(o-ethoxyphenoxy)ethylamino]propyl}-2-methoxybenzenesulfonamide hydrochloride: hereinafter, referred to as YM-12617) in plasma after orally administering the controlled-release pharmaceutical formulation of this invention as tablets to humans and

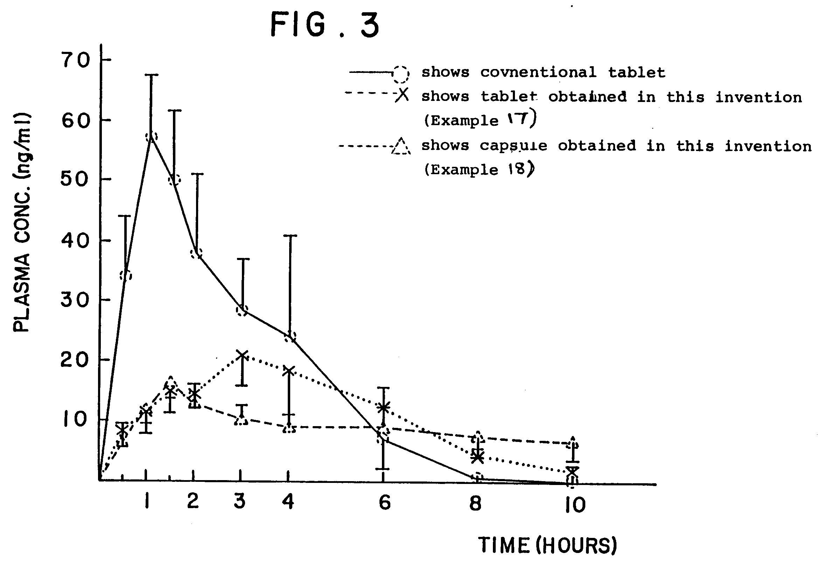

Fig. 3 is a graph showing the change with the passage of time of the concentration of a physiologically active substance (YM-12617) after orally administering the controlled-release pharmaceutical formulation of this invention as capsules to beagle dogs.

The granulation product (active substance-containing granules) of this invention may be water-permeable but is not substantially disintegrated (i.e., scarcely disintegrates or does not disintegrate for at least a few hours) in the gastrointestinal tract. Also, the pharmaceutical formulation of this invention has a high physical strength and the product is rarely crushed when formed into tablets by compression. Furthermore, by properly selecting an enteric coating agent and properly controlling the compounding ratio thereof in the preparation of the granulation product (active substance-containing granules), a granulation product having the desired dissolving characteristics can be obtained.

The purpose of the crystalline cellulose carrier is to provide, on granulation, cohesive units (granules) containing the active substance. The material used as carrier is capable of forming a structured particle of the formulation. The amount of carrier is at least 50% by weight, preferably at least 70% by weight based on the solids weight of the granules.

The release controlling agent in this invention is involved as a binding agent for granulation. Suitable water-insoluble macromolecular materials for use in this agent include acrylic acid series polymers, acrylic acid series copolymers, and cellulose derivatives such as ethyl cellulose,hydroxypropylmethyl cellulose phthalate,and hydroxypropylmethyl cellulose acetate succinate. The release controlling agent is suitably used in the form of an aqueous suspension, an aqueous emulsion, or a water-containing organic solvent solution. They are also commercially available as, for example, Eudragit L 30 D (Röhm Co., trade name; aqueous suspension of a methacrylic acid-ethyl acrylate copolymer), Eudragit E 30 D (aqueous suspension of an ethyl acrylate-methyl methacrylate copolymer), Aquacoat ECD-30 (aqueous suspension of ethyl cellulose), etc., which can be used as the release controlling agent as they are or diluted with water. Also, low-substituted hydroxypropyl cellulose (L-HPC) or ethyl cellulose can be used as an aqueous gel. Furthermore, water-insoluble polymers may be used as solution systems in a water-base mixed solvent containing an organic solvent.

The amount of release controlling agent is generally up to 30% (as solid component) or 50-150% (as aqueous liquid material) by weight based on the weight of the granules.

In this invention, for controlling the dissolving characteristics of an active substance, an alkaline earth metal salt ( or an alkali metal salt) of a higher fatty acid or an enterosoluble polymer may be added to the formulation.

The addition of the aforesaid material is effective when the physiologically active substance is a so-called micromedicament. Examples of the alkaline earth metal salt or alkaline metal salt of a higher fatty acid are magnesium stearate, and calcium stearate. Also, examples of the enterosoluble polymers are cellulose acetate phthalate, hydroxypropylmethyl cellulose phthalate, and a methacrylic acidmethyl methacrylate copolymer (Eudragit L, S). The compounding amount thereof is usually 1 to 15% by weight.
When such enterosoluble polymer is used a plasticiser such as PEG 6000, Tween 80 (trade name), and triacetin, may be added.

The compounding amount thereof is usually 0 to 15% by weight based on the weight of the release controlling agent (solid component).
In addition, an alkaline metal halide or an alkaline earth metal halide, such as sodium chloride, and calcium chloride can be used for the same purpose.

As described above, the release of the physiologically active substance can be controlled by selecting the kind of the release controlling agent and/or controlling the compounding amount of the alkaline earth metal salt (or alkaline metal salt) of a higher fatty acid or an enterosoluble polymer. According to the characteristics of the active substance, the release thereof can be also delayed by subjecting the active substance itself to a hydrophobic treatment. The hydrophobic treatment can be performed by microcapsulating the active substance by, for example a spray congealing method used wax. Examples of the wax which may be used for the purpose are hydrogenated vegetable oils such as hydrogenated castor oil.

There is no particular restriction on the physiologically active substance for use in this invention. The amount of the active substance is generally less than 30% by weight based on the weight of the granules.

In the test examples and examples described hereinbelow, 5-{2-[2-(o-ethoxyphenoxy)ethylamino]propy}-2-methoxybenzenesulfonamide hydrochloride (YM-12617) having a relatively low solubility in water (about 0.3 to 0.5%) was used as the active substance.

YM-12617 shows an α-blocking action and can be used for the treatment of hyperpiesia, cardiac insufficiency, lower urinary disease, etc.

The formulation of this invention may also contain any of the usual additives or excipients such as filler, and coloring agent.

Suitably, the mixture of active substance, carrier substance and optionally alkaline earth metal salt (or an alkali metal salt) of a higher fatty acid or an enterosoluble polymer is granulated after the addition of the above-described aqueous liquid material as a release controlling agent. Granulation can be performed by an agitation-type apparatus, a rotation-type apparatus, a centrifugal-type apparatus, a fluidized bed-type apparatus, or an apparatus of mixed types.

The size (diameter) of the granulation product (particles) is suitably 0.1 to 1.5 mm, preferably 0.2 to 1.0 mm.

The individual active-substance-containing particles can be formed into larger units such as tablets, capsules, granules, etc.

The active substance-containing particles of this invention have a high mechanical strength, and mostly do not disintegrate when formed into tablets.

They disperse widely in the gastrointestinal tract when they are administered to a living body. Also, they are water-permeable but do not substantially disintegrate; they gradually release active substance into the gastrointestinal tract, whereby a long controlled release can be attained. Also, intra- and inter-subject variations are very small and the physiological effects are excellent in reproducibility. Furthermore, the pharmaceutical formulation of this invention can be obtained by a simple and safe production process.

Tests and results showing dissolution characteristics and concentrations in plasma of the active substance of the controlled-release formulation of this invention are described below.

### (1) Dissolution Test:

### Test Procedure:

The test was performed by the paddle method of the 2nd dissolution test method in the Japan Pharmacopoeia. That is, the test was performed by a UV method or a liquid chromatograph method (shown below) at a rotation speed of the paddle of 150 r.p.m., using 500 ml of a 1st liquid (artificial gastric juice) of the Japanese Pharmacopoeia and 500 ml of a 2nd liquid (artificial intestinal juice) of the Japanese Pharmacopoeia, respectively. A sample was first tested in the 1st liquid for one hour followed by the test in the 2nd liquid for one hour.

### (i) UV Method

The pharmaceutical formulation obtained in each example shown below was used as a sample. The amount of each sample corresponding to 50 mg of YM-12617 was subjected to the aforesaid dissolution test, the dissolved liquid was filtered, and the active substance in the filtrate was determined at a detection wavelength of 278 nm.

### (ii) High Performance Liquid Chromatograph Method (HPLC Method):

The pharmaceutical formulation prepared in each example was used as a sample. The amount of the sample corresponding to 1 mg of YM-12617 was subjected to the aforesaid dissolution test, the dissolved liquid was filtered, and the active substance was determined under the following conditions.

### Operation Conditions:

Detector: Ultraviolet Spectrophotometer (Detection wavelength of 225 nm)
Column: In a stainless tube of about 4 mm inside diameter and about 150 mm length was packed about 5 µm of octadecylsililated silica gel (e.g., Nucleosil 5C18, trade name) as a packing agent
Column Temperature: About 35°C.
Mobile Phase: Mixture of 0.05N perchloric acid and acetonitrile (7 : 3).
Flow rate: Constant amount of 0.8 to 1.5 ml per minute.

### Test Results:

The results thus obtained are shown in Table 1.

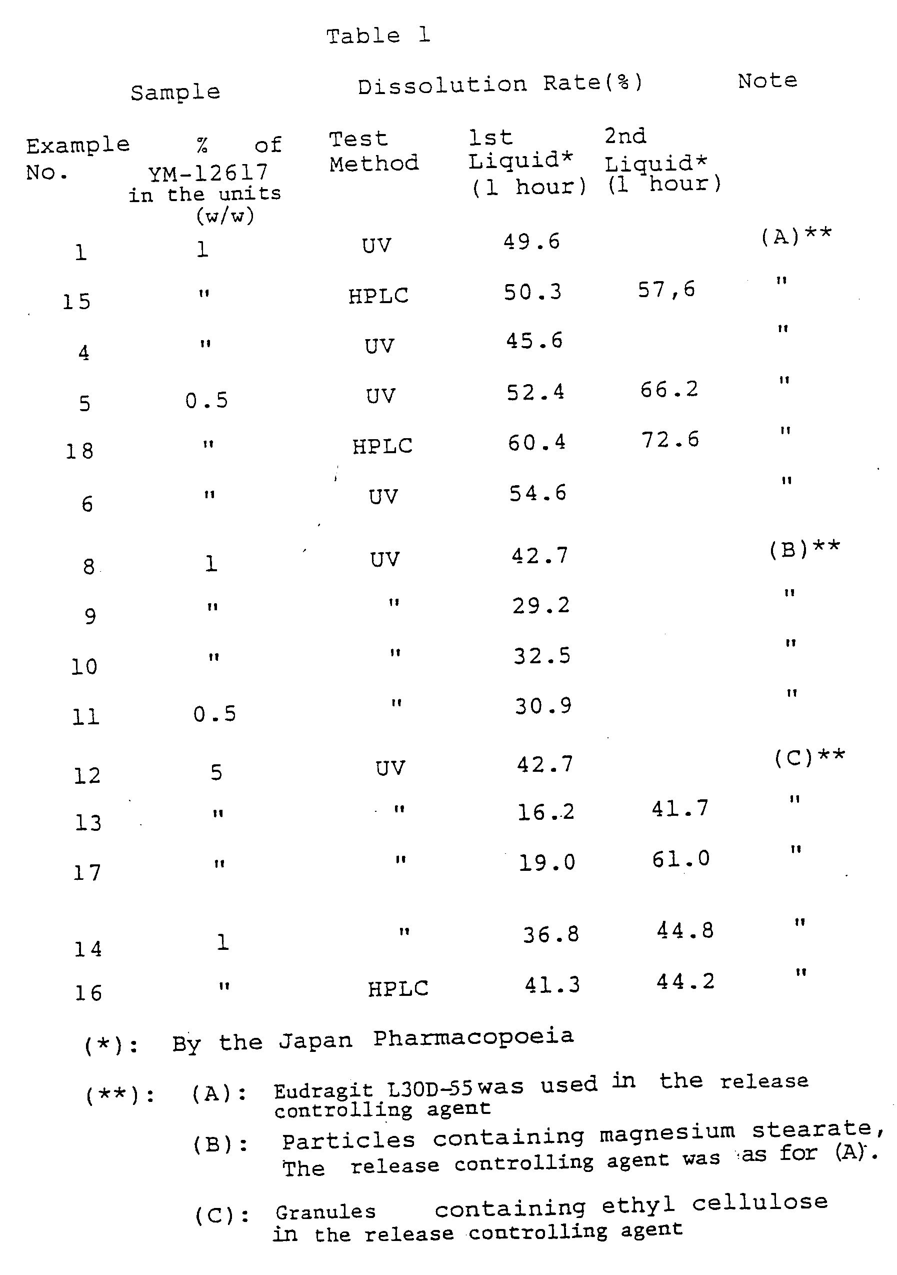

### (2) Absorption Studies following Oral Administration: (A)

(i) Tablets obtained in Example 15 were used as the sample of this invention and conventional tablets obtained in Refence Example 1 were used as a control . A dose corresponding to 1 mg of YM-12617 was orally administered to five adult male subjects, respectively, by a cross over method. Then, blood samples were withdrawn at definite time intervals and the concentration of the active substance in plasma was measured by the method shown below.
(ii) Determination Method of YM-12617 in Plasma:
   After adding 0.5 ml of an aqueous solution of internal standard substance (containing 0.5 µg of amosulalol hydrochloride) to 1.5 ml of plasma, 1 ml of a saturated aqueous solution of sodium hydrogencarbonate was added thereto and the active substance was extracted with 4 ml of ehtyl acetate. The ethyl acetate extract was further extracted with 2.5 ml of 0.4N hydrochloric acid. The hydrochloric acid layer thus obtained was adjusted to weak alkaline by the addition of 2 ml of a saturated aqueous solution of sodium hydrogencarbonate and then re-extracted with 4 ml of ethyl acetate.
   After adding 0.05 ml of an aqueous solution of 0.1M sodium hydrogencarbonate and 0.1 ml of an acetone solution of 500 µg of dansyl chloride, the ethyl acetate layer thus obtained was distilled under reduced pressure for 120 minutes at 35°C. After adding 4 ml of ether to the mixture, the organic layer thus formed was washed with 5 ml of water and then with 5 ml of an aqueous solution of 0.2 N hydrochloric acid. The solvent was distilled off from the organic layer, the residue thus formed was dissolved in 0.05 ml of a mixture of benzene and methanol (100 : 1), and using all of the solution the active substances was determined by a liquid chromatography under the following operation condition.
   The retention times for dansyl-YM-12617 and dansyl-amosulalol when the flow rate of the eluent was 1.4 ml/min. were 8.1 minutes and 12.5 minutes, respectively.
   Operation Condition
   Detector: Fluorescent Photometer (Excitation wavelength 365 n m , fluorescent wavelength 500 n m )
   Column: In a stainless tube of about 4 mm in inside dimater and about 250 mm in length was packed by about 5 µm of silica gel (e.g., Lichrosorb SI 100, trade name, made by Merck & Co., Ltd.) as a filler.
   Column Temperature: About 10°C
   Flow Rate : Constant flow rate of 1.2 to 1.9 ml per minute.
(iii) The results thus obtained are shown in Table 2, Table 3, and Fig. 1.
   In Fig. 1, 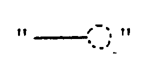 shows tablets obtained in Reference Example 1, and "-----X" shows tablets obtained in Example 15.

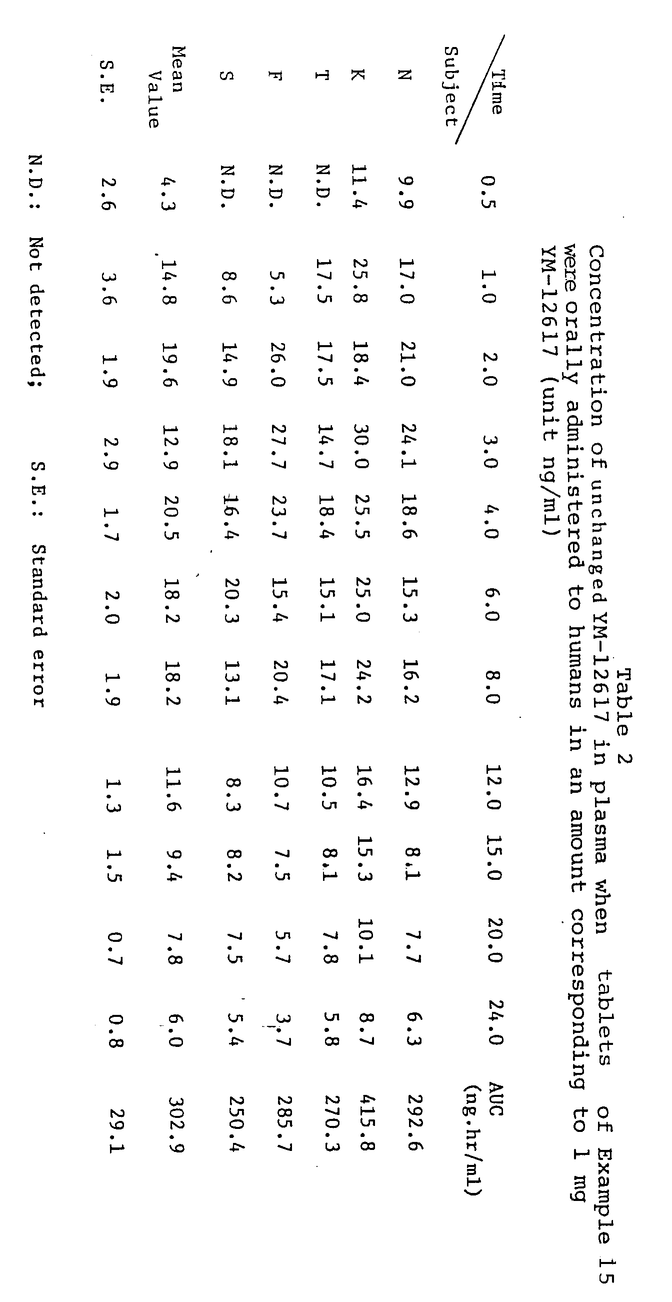

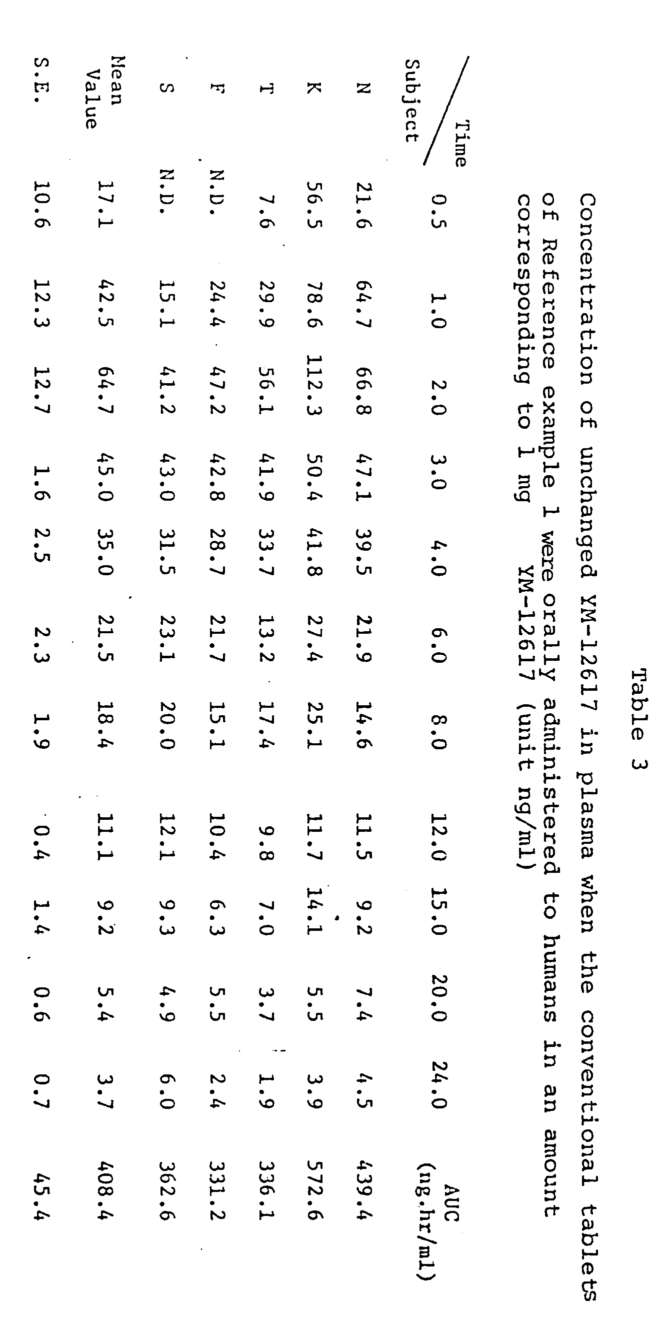

As is clear from Fig. 1, in the case of administering the tablets prepared in Example 15, the concentration pattern of the active substance in plasma was good and showed the following features.
a) The ratio of Cmax/Cmin is small, which shows long acting characteristics.
b) Intra-individual variation is small.

### (B)

(i) Tablets obtained in Example 16 were used as a sample of this invention and conventional tablets obtained in Reference Example 1 were used as a control. The amount of each sample corresponding to 1 mg of YM-12617 was orally administered to five adult male subjects, respectvely, by a cross over method. Blood was withdrawn at definite time intervals, and the concentration of the active substance in plasma was measured by the aforesaid method (A) (ii).
(ii) The results thus obtained are shown in Table 4, Table 5, and Fig. 2.
   In Fig. 2. 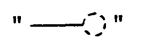 shows tablets obtained in Reference Example 1, and "------X "shows tablets obtained in Example 16.

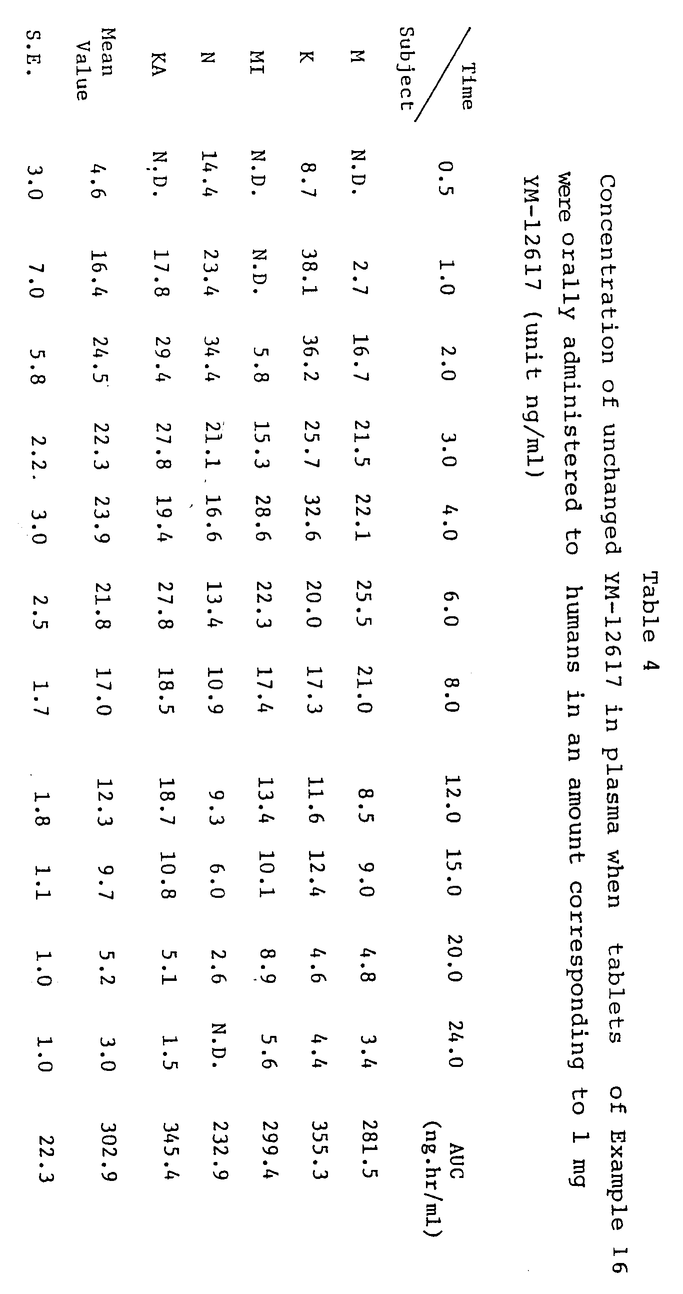

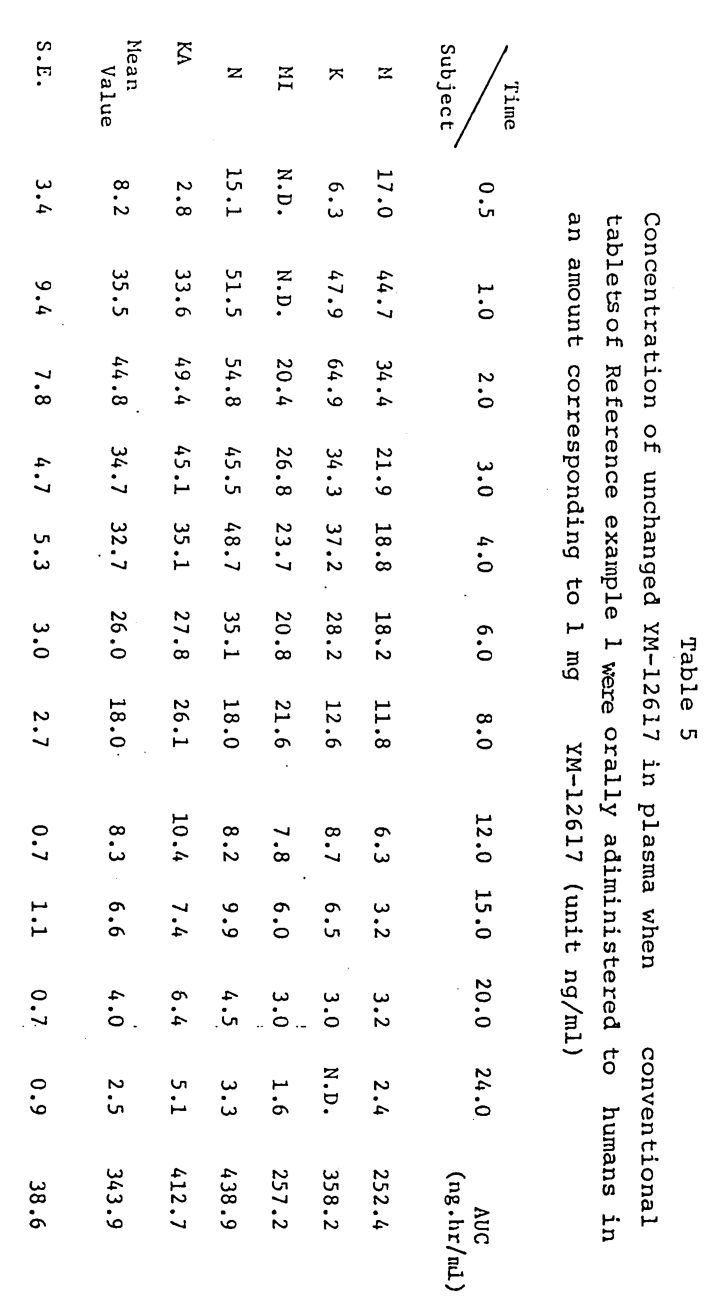

As is clear from Fig. 2, in the case of administrating the tablets prepared in Example 16, the concentration pattern of the active substance in plasma was good and showed the following features.
a) The ratio of Cmax/Cmin is small, which shows long acting characteristics.
b) Inter-individual variation is small.

### (C)

(i) Capsules obtained in Example 17 were used as the samples of this invention and conventional tablets obtained in Reference Example 2 were used as a control. The amount of each sample corresponding to 10 mg of YM-12617 was orally administered to six beagle dogs by a cross over method. Blood was withdrawn at definite intervals, and the concentration of the active substance in plasma was measured by the aforesaid method (A) (ii).
(ii) The results are shown in Fig. 3.
   In Fig. 3, 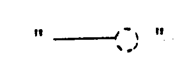 shows tablets obtained in Reference Example 2, and 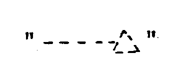 shows capsules obtained in Example 17.

As is clear from Fig. 3, in the case of administering capsules prepared in Example 17, the concentration patterns of the active substance in plasma are good and show the following features :
a) The ratio of Cmax/Cmin is small.
b) Inter-individual variation is small.

### (3) Relationship between dissolution characteristics and rotation speed in dissolution tests.

The rotation speed of the paddle in the dissolution test (1) described above was changed and the influence of the rotation speed on the dissolution rate was studied. (The active substance was determined by UV method.). The results thus obtained are shown in Table 6.

**Table 6**

| Example No. | % dissolved, JP, 1st Liquid*, After 1 Hour. | | | |
|---|---|---|---|---|
| | Rotation speed of the Paddle | | | |
| | 50 rpm | 100 rpm | 150 rpm | 200 rpm |
| 1 | 42.1 | 45.7 | 45.4 | 45.3 |
| 14 | 36.0 | 36.3 | 36.4 | 39.6 |

As is clear from Table 6, it can be seen that there is no change of the dissolution characteristics with change of rotation speed. Hence, the motion of the gastrointestinal tract in the living body scarcely influences the dissolution rate of the formulation of this invention.

### (4) Stability of Dissolution Characteristics with the Passage of Time:

The product obtained in each example was stored under severe conditions(shown in Table 7 below) for one month and then the dissolution test as in above-described test (1) was performed on the product. In this case, the determination of the active substance was performed by UV method.

The results thus obtained are shown in Table 7.

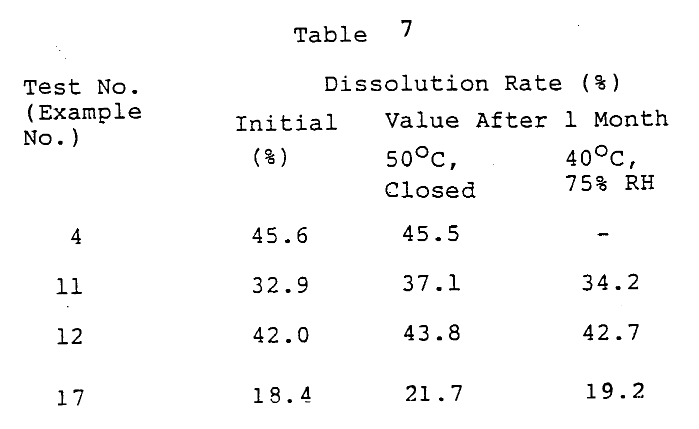

As is clear from the above results,
when the samples of this invention are stored under the severe conditions, the change in the dissolution characteristics is very small and thus, the formulations are stable to the passage of time.

### (5) Good Dissolution Reproducibility:

Three samples were prepared by the same manner as in Example 4 and the dissolution test as above was performed on each sample (the determination of the active substance was performed by UV method). The results thus obtained are shown in Table 8.

**Table 8**

| Sample No. (Example No.) | % dissolved JP, 1st Liquid* (1 hour) |
|---|---|
| Example 4 | 45.6 |
| 4 - 1 | 45.4 |
| 4 - 2 | 45.9 |
| 4 - 3 | 45.3 |

| | |
|---|---|
| (*): The 1st liquid in Tables 6 and 8 above is artificial gastric juice in the Japan Pharmacopoeia. | |

From the results shown in Table 8, it can be seen that the samples of this invention show good dissolution reproducibility.

Examples 1-17 of the invention will now be described below, followed by Reference Examples 1 and 2.

### Example 1 (Production of active substance-containing granules)

After sufficiently mixing 5 g of YM-12617 and 470 g of crystalline cellulose, a mixture of 83.3 g (25 g as solid component) of Eudragit L30D-55(Röhm Co.)and 500 g of water was added to the aforesaid mixture and the resultant mixture was granulated by a high-speed mixer. The granules obtained were spheres having particle sizes of 0.1 to 1.5 mm, mainly 0.2 to 1.0 mm.

### Examples 2 to 7

By following the same procedure as Example 1 using the formulae shown in Table 9 below, active substance-containing granules were prepared.

**Table 9**

| Formula (g) | Example No. | | | | |
|---|---|---|---|---|---|
| 2 | 3 | 4 | 5 | 6* | 7 |
| YM-12617 5 | 5 | 5 | 2.5 | 2.5 | 1.25 |
| Crystalline 445 Cellulose | 395 | 482.5 | 472.5 | 472.5 | 473.75 |
| Eudragit L30D-55 | | | | | |
| (Solid comp.) 166.6 (50) | 333.3 (100) | 41.7 (12.5) | 83.3 (25) | 83.3 (25) | 83.3 (25) |

| | | | | | |
|---|---|---|---|---|---|
| (*): Centrifugal fluidized bed granulator was used. | | | | | |

### Example 8

After sufficiently mixing 5 g of YM-12617, 420 g of crystalline cellulose, and 50 g of magnesium stearate, a mixture of 83.3 g (25 g as solid component) of Eudragit L30D-55 and 500 g of water was added to the aforesaid mixture and the resultant mixture was kneaded and granulated by a centrifugal fluidized bed granulator. The granules obtained were spheres having particle sizes of 0.1 to 1.5 mm, mainly 0.2 to 1.0 mm.

### Examples 9 to 11

By following the same procedure as Example 8 using the formulae shown in Table 10, active substance-containing granules were prepared.

**Table 10**

| Formula (g) | Example No | | |
|---|---|---|---|
| | 9 | 10 | 11 |
| YM-12617 | 5 | 5 | 2.5 |
| Crystalline Cellulose | 460 | 445 | 462.5 |
| Magnesium Stearate | 10 | 25 | 10 |
| Eudragit L30D-55 (Solid component) | 83.3 (25) | 83.3 (25) | 83.3 (25) |

### Example 12

After sufficiently mixing 20 g of YM-12617, 300 g of crystalline cellulose, and 80 g of ethyl cellulose, 230 g of a mixed solvent of ethanol and water (8 : 2 mixing ratio) was added to the mixture and the resultant mixture was granulated by a high-speed mixer. The particle size of the particles thus obtained were same as described above.

### Example 13

By following the same procedure as Example 12 and granulating the mixture by means of an ultra high-speed mixer, granules were prepared. The particle sizes of the granules were same as above.

### Example 14

After melting 80 g of hydrogenated castor oil, 10 g of YM-12617 and 30 g of low-substituted hydroxypropyl cellulose were dispersed in the melt and the resultant mixture was granulated by a spray congealing method. After sufficiently mixing 60 g (5 g YM-12617) of the granulated product thus obtained and 440 g of crystalline cellulose, 500 g of water was added to the mixture and the resultant mixture was granulated by means of a centrifugal fluidized bed granulator. The particle sizes of the granules thus obtained were same as above.

### Example 15

To 20 g of the particles (active substance-containing granules obtained in Example 1 were added 44.9 g of lactose, 20 g of starch, 9.7 g of crystalline cellulose, 5 g of CMC-Ca, and 0.5 g of magnesium stearate and tablets were prepared using the mixture thus obtained by a conventional method (one tablet of 100.1 mg contained 0.2 mg of YM-12617).

### Examples 16 and 17

By following the same procedure as Example 15 using the formulae shown in Table 11 below, pharmaceutical formulations in tablet and capsule form were prepared.

**Table 11**

| Formula, etc. | Example No. | |
|---|---|---|
| | 16 | 17 |
| Granules | 20 g* | 50 g*** |
| Lactose | 46.5 g | 50 g |
| Starch | 28 g | - |
| Magnesium Stearate | 0.5 g | - |
| Crystalline Cellulose | - | - |
| CMC-Ca | - | - |
| Hydrogenated Oil | - | - |
| Formulation | Tablet | Capsule^{4*} |
| Weight of one formulation | 100 mg | 100 mg |

| | | |
|---|---|---|
| (*): The granules obtained in Example 14 | | |
| (***): The granules obtained in Example 13 | | |
| (*4): Encapsulated by a conventional method. | | |

### Example 18

After sufficiently mixing 40 g of the granules obtained in Example 5, 24 g of lactose, 34.54 g of crystalline cellulose, 12 g of low-substituted hydroxypropyl cellulose, and 3 g of corn starch, 40 g of 10% corn starch paste was added to the mixture and the resultant mixture was granulated by a conventional method. Then, 2.4 g of a hydrogenated oil and 0.06 g of calcium stearate were added to the granules thus obtained and tablets were manufactured using the resultant mixture by a conventional method (one tablet of 120 mg contained 0.2 mg of YM-12617).

### Example 19

After sufficiently mixing 5 g of YM-12617 and 467.5 g of crystalline cellulose, a mixture obtained by adding 414.2 g of water and 2.5 g of PEG 6000 to 83.3 g (25 g as solid component ) of Eudragit L30D-55 (trade name) was added to the aforesaidmixture and the resultant mixture was granulated by a high-speed mixer. The granules were spheres having particle sizes of 0.1 to 1.5 mm, mainly 0.2 to 1.0 mm.

### Reference Examples 1 and 2

Conventional tablets were prepared using the formulae shown in Table 14 below.

**Table 14**

| Formula | Reference Example No. | |
|---|---|---|
| | 1 | 2* |
| YM-12617 | 0.2 g | 2.5 g |
| Lactose | 66.7 g | 63.0 g |
| Starch | 28.6 g | - |
| " (for paste) | 3.5 g | - |
| Magnesium Stearate | 1.0 g | 1.0 g |
| Corn Starch | - | 30.0 g |
| " (for paste) | - | 3.5 g |
| tablet | 100 mg | 100 mg |

| | | |
|---|---|---|
| (*): Prepared by fluidized bed granulator. | | |

## Claims

1. A method for making granules for a controlled-release pharmaceutical formulation, the method comprising granulating, to give granules of a diameter of from 0.1 to 1.5 mm, a mixture of (a) 5-{2-[2-(o-ethyoxyphenoxy)ethylamino]propyl}-2-methoxybenzene-sulfonamide hydrochloride, (b) crystalline cellulose carrier in an amount of at least 50 solids wt.% of the mixture and (c) release control agent comprising water and water-insoluble macromolecular substance, the release control agent being in the form of an aqueous emulsion or suspension or gel of said macromolecular substance or in the form of a solution of said macromolecular substance in an aqueous organic solvent, and said macromolecular substance being selected from acrylic acid series polymers and copolymers and cellulose derivatives, the amount of the release controlling agent being up to 30% (as solid component) or 50-150% (as aqueous liquid material) based on the weight of the granules.

2. A pharmaceutical sustained release formulation comprising granules of a diameter of from 0.1 to 1.5 mm obtainable by adding release control agent comprising water and water-insoluble macromolecular substance to a mixture of 5-{2-[2-(o-ethyoxyphenoxy)ethylamino]propyl } -2-methoxybenzene-sulfonamide hydrochloride and crystalline cellulose carrier and granulating the resultant mixture, the crystalline cellulose carrier constituting at least 50% by weight of the solids in the mixture, the release control agent being in the form of an aqueous emulsion or suspension or gel of said macromolecular substance or in the form of a solution of said macromolecular substance in an aqueous organic solvent, and said macromolecular substance being selected from acrylic acid series polymers and copolymers and cellulose derivatives, the amount of the release controlling agent being up to 30% (as solid component) or 50-150% (as aqueous liquid material) based on the weight of the granules.

3. A formulation according to claim 2 in tabletted or capsuled form.

## Patentansprüche

1. Verfahren zur Herstellung von Körnern zur gesteuerten Freisetzung von pharmazeutischen Zubereitungen, wobei das Verfahren umfaßt das Granulieren zu Körnern mit einem Durchmesser von 0,1 bis 1,5 mm eines Gemisches aus (a) 5-{2-[2-(o-Ethoxyphenoxy)ethylamino]propyl}-2-methoxybenzolsulfonamid-hydrochlorid, (b) kristallinem Celluloseträger in einer Menge von mindestens 50 Gew.-% Feststoffen, bezogen auf das Gemisch und (c) einem Mittel zur Steuerung der Freisetzung, umfassend Wasser und (eine) wasserunlösliche makromolekulare Substanz(en), wobei das Mittel zur Steuerung der Freisetzung in Form einer wäßrigen Emulsion oder Suspension oder eines Gels der makromolekularen Substanz oder in Form einer Lösung der makromolekularen Substanz in einem wäßrig organischen Lösungsmittel vorliegt und die makromolekulare Substanz ausgewählt ist aus Acrylsäurepolymeren und -copolymeren und Cellulosederivaten, wobei die Menge an Mittel zur Steuerung der Freisetzung bis zu 30 % (als Feststoff) oder 50 bis zu 150 % (als wäßriges flüssiges Material), bezogen auf das Gewicht der Körner, ausmacht.

2. Pharmazeutische Zubereitung mit verzögerter Wirkstoffabgabe, umfassend Körner mit einem Durchmesser von 0,1 bis 1,5 mm, erhältlich durch Zugabe eines Mittels zur Steuerung der Freisetzung, umfassend Wasser und (eine) wasserunmischbare makromolekulare Substanz(en) zu einem Gemisch aus 5-{2-[2-o-Ethoxyphenoxy)ethylamino]propyl}-2-methoxybenzol-sulfonamid-hydrochlorid und kristallinem Celluloseträger und Granulieren des erhaltenen Gemisches, wobei der kristalline Celluloseträger mindestens 50 Gew.-% der Feststoffe in dem Gemisch ausmacht und das Mittel zur Steuerung der Freisetzung in Form einer wäßrigen Emulsion oder Suspension oder eines Gels der makromolekularen Substanz oder in Form einer Lösung der makromolekularen Substanz in einem wäßrig organischen Lösungsmittel vorliegt und die makromolekulare Substanz ausgewählt ist aus Acrylsäurepolymeren und -copolymeren und Cellulosederivaten, wobei die Menge an Mittel zur Steuerung der Freisetzung bis zu 30 % (als Feststoff) oder 50 bis 150 % (als wäßriges flüssiges Material), bezogen auf das Gewicht der Körner, ausmacht.

3. Zubereitung nach Anspruch 2 in Form von Tabletten oder Kapseln.

## Revendications

1. Un procédé de fabrication de granulés pour une formulation pharmaceutique à libération contrôlée, le procédé comprenant une granulation pour produire des granulés d'un diamètre de 0,1 à 1,5 mm, un mélange de (a) chlorhydrate de 5-{2-[2-(o-éthyoxyphénoxy)éthylamino]propyl}-2-méthoxybenzène-sulfonamide, (b) un support en cellulose cristalline en quantité égale à au moins 50 % en poids des solides du mélange et (c) un agent de contrôle de libération comprenant de l'eau et une substance macromoléculaire insoluble dans l eau, l'agent de contrôle étant sous la forme d'une émulsion aqueuse ou d'une suspension ou d'un gel de ladite substance macromoléculaire ou sous la forme d'une solution de ladite substance macromoléculaire dans un solvant organique aqueux, et ladite substance macromoléculaire étant choisie parmi des séries de polymères et de copolymères d'acide acrylique et des dérivés de cellulose, la teneur en agent de contrôle de libération allant jusqu'à 30% (en tant que composé solide) ou 50-150 % (en tant que matériau liquide aqueux) par rapport au poids des granulés.

2. Une formulation pharmaceutique à libération prolongée comprenant des granulés d'un diamètre de 0,1 à 1,5 mm pouvant être obtenus par addition d'un agent de contrôle de libération comprenant de l'eau et une substance macromoléculaire insoluble dans l'eau à un mélange de chlorhydrate de 5-{2-[2-(o-éthyoxyphénoxy)éthylamino]propyl}-2-méthoxybenzène-sulfonamide et d'un support de cellulose cristalline et par granulation du mélange résultant, le support de cellulose cristalline constituant au moins 50 % en poids des solides dans le mélange, l'agent de contrôle de libération étant sous la forme d'une émulsion aqueuse ou d'une suspension ou d'un gel de ladite substance macromoléculaire ou sous la forme d'une solution de ladite substance macromoléculaire dans un solvant organique aqueux, et ladite substance macromoléculaire étant choisie parmi des séries de polymères et de copolymères d'acide acrylique et des dérivés de cellulose, la teneur en agent de contrôle de libération allant jusqu'à 30% (en tant que composant solide) ou 50-150 % (en tant que matériau liquide aqueux) par rapport au poids des granulés.

3. Une formulation conforme à la revendication 2 sous forme de comprimé ou de capsule.
